# EUROPEAN PATENT APPLICATION

(11) **EP 4 679 281 A1**
(43) Date of publication of application: **14.01.2026**
(21) Application number: 25188319.5
(22) Date of filing: 09.07.2025
(51) Int. Cl.: G06F 16/242, G06F 16/2457

(54) **SYSTEMS AND METHODS FOR ARTIFICIAL INTELLIGENCE DRIVEN CLINICAL TRIAL PROTOCOL DATA RETRIEVAL AND AUGMENTATION**

(30) Priority: 10.07.2024 US 202418769143
(71) Applicant: Medidata Solutions, Inc., New York, NY 10014 (US)
(72) Inventor: NOUIRA, Marouane, New York, 10014 (US)
(74) Representative: D Young & Co LLP

(57) **Abstract**

Disclosed are methods and systems for artificial intelligence - driven clinical trial protocol data retrieval and augmentation. A natural language user request is received via a user interface. The user request is combined with contextual data to produce a prompt for a large language model. The prompt is input to the model to produce a model response including a database query, in a database query language, and metadata. Clinical trial protocol data is retrieved from a first database based on the database query. Application programming interface (API) requests are generated based on the database query and/or the metadata. API calls are performed using the generated API requests to obtain clinical trial metrics. A response to the user request is generated based on the retrieved protocol data and the metrics.

## Description

### FIELD AND BACKGROUND

The present disclosure generally relates to artificial intelligence - driven clinical trial protocol data retrieval and augmentation.

Clinical studies, i.e., clinical trials, tend to have diverse requirements - every clinical trial is unique and so are its requirements. A study protocol is an essential document for defining a clinical trial and its requirements. The study protocol is a text document containing information on overall study design, objectives, patient cohorts, and procedures, etc. Traditionally, the process of drafting a study protocol requires medical professionals, often doctors working in pharmaceutical companies, to manually write out these protocols as a formal document. The process is inefficient at best and these professionals often resist using technology to assist in generating study protocols.

Conventional clinical trial design software, such as proprietary software from clinical trial management system vendors and open-source solutions like OpenClinica or REDCap, focus on data capture and management rather than proactive protocol design. These tools typically require substantial manual input and do not utilize artificial intelligence (AI) to dynamically suggest improvements or predict outcomes based on historical data. A further shortcoming is that they are generally not equipped to offer real-time metrics or predictive analytics.

Some conventional systems integrate AI to optimize certain aspects of trials, such as patient recruitment or site selection. Examples include IBM Watson for Clinical Trial Matching or Antidote's Match, which focus on matching patients to trials using AI. However, while these tools utilize AI to an extent, they do not provide comprehensive assistance in designing the trial protocol itself. Their focus is narrower, often limited to improving recruitment or operational aspects post-protocol finalization.

Conventional protocol repositories and analytical tools, such as clinicaltrials.gov, provide a database of registered clinical trial protocols. Some analytical tools offer insights based on this data, but they do not guide the protocol design process. Moreover, such repositories are static and primarily for reference. They do not offer interactive tools for protocol creation or modification based on predictive modeling.

### SUMMARY

Particular aspects are set out in the appended independent claims. Various optional embodiments are set out in the dependent claims.

Disclosed embodiments provide sophisticated tools for clinical trial design and management. This includes technology for leveraging generative artificial intelligence (AI) to analyze historical clinical trial protocols and other relevant data. The technology assists in creating efficient and effective clinical study protocols by suggesting optimized procedures, estimating key trial metrics, and enhancing decision-making processes in clinical trial management.

Disclosed embodiments relate to healthcare informatics, in that, beyond the application to clinical trials, the technology could be useful in other areas of healthcare data management, such as patient treatment plans, hospital resource allocation, and public health studies.

Disclosed embodiments relate to educational tools. In academic or training settings, the technology could be used to simulate clinical trial design scenarios, helping students and new researchers understand the complexities of trial design and the impact of various factors on trial outcomes.

In disclosed embodiments, a model, e.g., a generative artificial intelligence (AI) model, is developed and fine-tuned to specialize in clinical study metrics, enhancing its capability to act as an expert system for clinical trial protocol generation. This includes training the model to understand and interpret the nuances of clinical data and trial outcomes, enabling it to generate and optimize trial protocols based on learned patterns and predictive analytics. The fine-tuning process helps ensure the model provides accurate, context-aware suggestions that reflect the complexities and specific requirements of clinical trials.

Disclosed embodiments provide an AI-based agent that serves as the primary interface between the end-users and the system. The agent is configured to direct user queries to appropriate systems for processing and response. While the agent's main interaction will be with the model for immediate adjustments to the trial protocols based on user inputs, the agent is also configured to interface with other clinical trials data systems. For example, if a user proposes a new inclusion criterion, this information is processed by the model to update the trial protocol. The same information may be relayed to a cost estimator service, enabling it to provide an updated budget analysis which is then displayed to the user. This multi-faceted approach helps ensure that the agent provides a holistic service, thereby enhancing user experience and operational efficiency.

In one aspect, the disclosed embodiments provide methods, systems, and computer-readable media for artificial intelligence - driven clinical trial protocol data retrieval and augmentation. The method includes receiving a natural language user request via a user interface. The method further includes combining the user request with contextual data to produce a prompt for a large language model. The method further includes inputting the prompt to the model to produce a model response comprising a database query, in a database query language, and metadata. The method further includes retrieving clinical trial protocol data from at least a first database based at least in part on the database query. The method further includes generating one or more application programming interface (API) requests based at least in part on one or more of: the retrieved protocol data and the metadata. The method further includes performing one or more API calls using the generated API requests to obtain one or more clinical trial metrics. The method further includes generating a response to the user request based at least in part on the retrieved protocol data and the metrics. The method further includes outputting the response to the user interface.

Embodiments may include one or more of the following features, alone or in combination.

In the combining of the user request with the contextual data, the contextual data may comprise a schema for the first database. The contextual data may comprise domain knowledge definitions, including one or more of the following: electronic data capture query definition and patient burden index definition. The contextual data may comprise an output format definition. The contextual data may comprise persona instructions for the model.

In the inputting the prompt to the model, the database query language may be SQL. The first database may store historical clinical trial protocol data and, in the retrieving clinical trial protocol data, the first database may be accessed via an API call to a publicly available uniform resource locator (URL). The retrieving of the clinical trial protocol data may further comprise retrieving additional clinical trial protocol data from a second, proprietary database; and correlating the clinical trial protocol data retrieved from the first database and the second database using respective national clinical trial (NCT) numbers. In performing said one or more API calls using the generated API requests, said one or more API calls may be made to one or more of the following: a screening failure predictor, a budget calculator, and a patient burden index calculator.

The method may further include parsing the model response to extract the database query; executing the database query against said at least first database; and replacing at least a portion of the metadata with the retrieved protocol data to produce an augmented model response. The method may further include parsing the augmented model response to extract the metadata; and comparing variables of the extracted metadata to variables of the APIs.

The inputting of the prompt to the model to produce the model response comprising the database query and metadata may use a zero-shot learning training process. The method may further include iteratively refining the prompt to improve the performance of the model. The inputting of the prompt to the model to produce model response comprising the database query and metadata may use a few-shot learning training process. The method may further include inputting a small number of manually-labeled examples to train the model. The method may further include performing fine tuning of the model using a curated dataset which is continuously updated. The method may further include receiving, after said outputting, a user rating of the response via the user interface; and performing training of the model based at least in part on the user rating.

### BRIEF DESCRIPTION OF THE DRAWINGS

Fig. 1 is a diagram depicting a system for artificial intelligence - driven clinical trial protocol data retrieval and augmentation, according to disclosed embodiments.
Fig. 2 is a diagram depicting historical clinical trial data extraction and correlation between a first, publicly-accessible database and a second, proprietary database.
Figs. 3A-3B depict generating a system prompt from a user request and a response obtained when the system prompt is input to a large language model (LLM).
Fig. 4 depicts a data model schema and corresponding database table.
Fig. 5 is a diagram depicting a validation and training system in which user-validated pairs of user requests and database queries are stored in a database to be used for training.
Fig. 6 depicts a method of artificial intelligence - driven clinical trial protocol data retrieval and augmentation, in accordance with disclosed embodiments.

Where considered appropriate, reference numerals may be repeated among the drawings to indicate corresponding or analogous elements. Moreover, some of the blocks depicted in the drawings may be combined into a single function.

### DETAILED DESCRIPTION

In the following detailed description, numerous specific details are set forth in order to provide a thorough understanding of the disclosed teachings. However, it will be understood by those of ordinary skill in the art that the disclosed teachings may be practiced without these specific details. In other instances, well-known methods, procedures, components, and circuits have not been described in detail so as not to obscure the presently taught approaches.

Disclosed embodiments provide an AI-driven protocol assistant that leverages natural language processing and machine learning (i.e., artificial intelligence) to automate the generation and optimization of clinical trial protocols, thereby significantly reducing manual effort and errors. Disclosed features include real-time optimization capabilities, allowing the system to adapt protocols based on new data and evolving research goals. In embodiments, the system uses advanced data mining techniques to analyze historical trial data, thereby enhancing the predictive accuracy of new protocols.

In disclosed embodiments, predictive analytics estimate key trial metrics to enable proactive adjustments. For example, a strategic recommendations engine may provide targeted advice which is tailored to specific trial goals, such as increasing diversity or reducing costs. Additionally, disclosed embodiments provide a user-friendly interface, making sophisticated trial design accessible to a broader range of research personnel. Collectively, these features address the inefficiencies and limitations of current trial design processes by enabling more informed, dynamic, and data-driven decision-making.

The disclosed systems and methods address a number of specific technical problems.

One technical problem addressed by disclosed embodiments is lack of dynamic optimization. Conventional tools typically do not dynamically suggest protocol adjustments based on real-time data inputs or evolving research landscapes. In contrast, the approaches described herein provide ongoing optimization suggestions as new data becomes available or as user-defined parameters change (e.g., increasing diversity and reducing costs).

Another technical problem addressed by disclosed embodiments is the insufficient use of historical data in conventional approaches. Conventional clinical trial design methods may underutilize the vast amounts of data available from past trials. The approaches disclosed herein, in contrast, leverage historical protocols and outcomes to inform better trial design decisions, thus enhancing the predictive power and relevance of the protocols.

Another technical problem addressed by disclosed embodiments is inadequate capability for the prediction of trial metrics. Conventional tools typically do not predict trial outcomes or metrics such as patient burden index, query rates, enrollment success, etc. In disclosed embodiments, an AI-driven assistant uses historical data and statistical models to forecast these metrics, allowing researchers to modify the protocol proactively to address potential issues before the trial begins.

Another technical problem addressed by disclosed embodiments is limited decision support for enhancing user-defined priorities. Specifically, conventional approaches lack supportive tools that can provide suggestions tailored to specific goals, such as increasing participant diversity or managing clinical trial costs effectively. The disclosed embodiments use AI-driven tools to suggest clinical trial sites and countries, as well as specific trial procedures that help meet the strategic goals.

Among other advantages, the disclosed embodiments provide enhanced efficiency in generating clinical trial protocols by automating the generation and optimization of the protocols. The features disclosed herein significantly reduce the time required to draft and revise clinical trial documents. This allows research teams to allocate more resources to other critical aspects of trial preparation and execution. Furthermore, the real-time optimization features disclosed herein allow for quick adjustments to protocols in response to new data or changes in trial objectives. This capability helps to ensure that clinical trial designs can rapidly adapt to new information, significantly speeding up the iterative process of trial design compared to conventional approaches that require manual revisions.

The disclosed embodiments provide, in effect, a strategic recommendations engine, which provides targeted suggestions to improve specific aspects of a clinical trial, such as increasing participant diversity and reducing operational costs. These recommendations are based on deep data analysis and predictive modeling, offering more sophisticated and nuanced insights than conventional tools. Furthermore, by integrating predictive analytics, the disclosed features not only provide forecasts of clinical trial outcomes, but also provide metrics that help anticipate potential challenges in patient enrollment, retention, and other critical areas. This proactive approach allows for better planning and management of the trial, minimizing risks and enhancing overall trial success.

The disclosed embodiments achieve further advantages by leveraging machine learning to analyze historical data. This helps ensure that the protocols generated are not only based on current best practices, but also informed by past successes and failures. This historical insight enhances the accuracy of the protocol recommendations, thereby reducing the likelihood of costly errors or oversights.

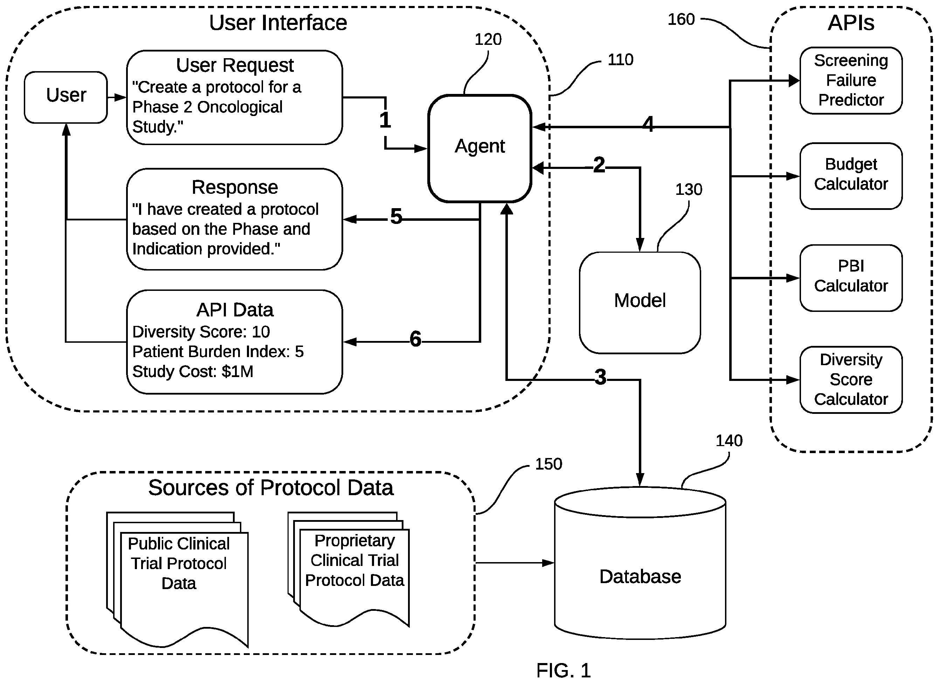

Fig. 1 is a diagram depicting a system for artificial intelligence - driven clinical trial protocol data retrieval and augmentation, according to disclosed embodiments. The clinical trial protocol data, e.g., historical clinical trial protocol data, may be used as a basis for producing a clinical trial protocol (or "study protocol") for a future clinical trial. The study protocol is a text document containing information on overall study design, objectives, patient cohorts, and procedures, etc. For example, a study protocol may include: the background and rationale, the objectives (e.g., primary, secondary, and exploratory objectives), type (e.g., randomized, double-blind, placebo-controlled), the number of subjects, the duration and phases, inclusion and exclusion criteria, assessments and procedures (e.g., details of medical examinations and laboratory tests, schedules of events or visits, etc.), treatment of subjects (e.g., the investigational product, dosage, and method of administration), procedures for monitoring drug efficacy and safety, definitions and procedures for adverse events and serious adverse events, data management and statistical methods, quality control and quality assurance, ethical considerations, publication and data sharing policies, references to relevant scientific literature, and various types of forms (e.g., informed consent, questionnaires, surveys, etc.).

In the disclosed system, a user interface 110 is accessed by a user to input a natural language user request to obtain clinical trial protocol data. For example, the user request could be: "create a protocol for a Phase 2 Oncological study," as depicted in the diagram. A user request may also be configured to retrieve other types of information from a variety of external sources. For example, the user may require estimates of various clinical trial metrics, such as diversity score, patent burden index (PBI), and study cost estimates (i.e., budget).

In embodiments, the user interface 110 may be implemented as an agent 120, which is a software and/or hardware subsystem which is configured to engage in natural language communication with the user and to coordinate the retrieval of the clinical trial data according to the methods disclosed herein. As discussed in further detail below, the agent 120 functions to intelligently orchestrate the interaction between user inputs, the historical database 140 of clinical trial data, and external application programming interfaces (APIs). A primary role of the agent 120 is to help ensure that the generated clinical trial protocols are comprehensive, accurate, and up-to-date, leveraging both internal and external data sources as needed.

An "agent," e.g., agent 120, in the context of a hardware and software-based system is a conceptual model that encapsulates particular functionalities of the overall software system. Identifying and describing an "agent" is a way to abstract and compartmentalize specific tasks or functions, making the system easier to understand and manage. However, it is important to note this is only a model or a framework for understanding the operation of the system. In practice, particular functions attributed to an agent may indeed be performed by other parts of the system. This situation arises due to the interconnected nature of software systems, where different modules or components often work together to achieve a task. Therefore, while an agent helps to visualize and explain the functionality of the disclosed system, the actual execution may involve various parts of the software outside of the "agent."

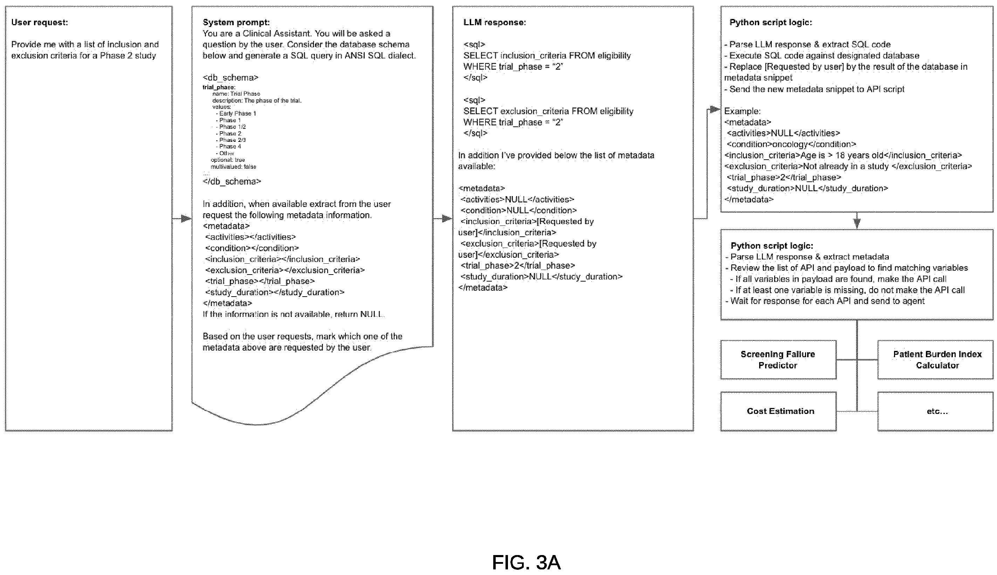

The user request is received by the agent 120, as indicated by the line labeled "1" in the figure. As discussed in further detail below, the agent 120 combines the user request with contextual data (see, e.g., discussion of Figs. 3A-3B) to produce a prompt for an artificial intelligence - based model 130, such as a large language model (LLM). The agent 120 inputs this formulated prompt to the model 130 (as indicated by the line labeled "2") to produce a model response which includes a database query, in a database query language, e.g., Structured Query Language (SQL), and associated metadata. The agent 120 uses the database query to retrieve clinical trial protocol data from the historical database 140 (as indicated by the line labeled "3").

In disclosed embodiments, the agent 120 is pre-configured with knowledge of all available external APIs 160 it can execute against, i.e., to which it can direct an API call. Each API 160 is documented with its endpoint, required payload structure (e.g., variables to be passed), and expected response format. Based on the database query and/or the metadata produced by the model 130, the agent 120 generates API requests and performs API calls using the generated API requests (as indicated by the line labeled "4") to obtain API data, such as clinical trial metrics. In embodiments, the API calls may be made made to one or more of the following APIs (160): a screening failure predictor, a budget calculator, and a patient burden index calculator.

In the context of the disclosed software system, an "external API" is typically used to enable communication or interaction with another software system or service. It provides a set of rules and protocols for how the software systems should interact, allowing them to exchange data and functionalities seamlessly. In some cases, an external API might be part of a separate system located on the same server, a different server, or even on a cloud-based platform. It could be developed by the same organization for internal use, or it could be provided by a third-party software system. However, the term "external" does not necessarily mean that the external API is located remotely or that it is part of a third-party system. It may simply mean that the API is outside of the main software system in question, operating independently, and can be leveraged to enhance or extend the functionality of the core system. Even if the API is part of the same overall system or developed by the same organization, if it is designed to operate independently or in conjunction with other systems, it may be referred to as an "external API."

The agent 120 generates a response to the user request based at least in part on the retrieved protocol data and the metrics outputs the response to the user interface 110. The response may include a natural language response output to the user (as indicated by the line labeled "5") and the API data resulting from the API calls (as indicated by the line labeled "6"). In embodiments, these outputs may be combined in a single response to the user, e.g., a single natural language response.

In the example depicted, the response states: "I have created a protocol based on the Phase and Indication provided." The created protocol may be in the form of text which follows the response and/or in the form of a document or spreadsheet provided as a separate file. The file may be sent to the user and/or made available to download. In some cases, the response may include a link to access the created protocol.

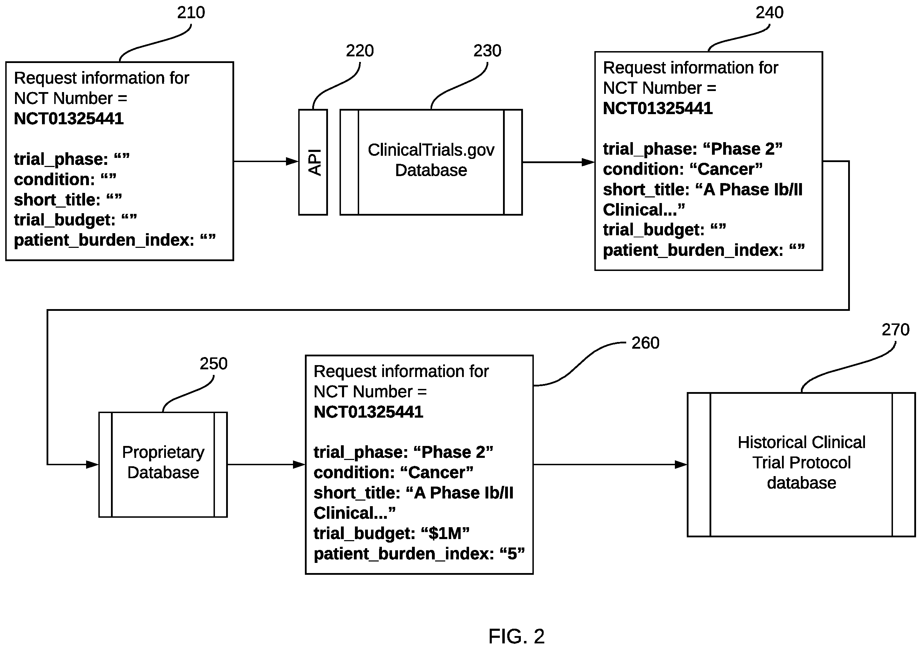

Fig. 2 is a diagram depicting historical clinical trial data extraction and correlation between a first, publicly-accessible database and a second, proprietary database. In embodiments, the first database stores historical clinical trial protocol data and may be accessed via an API call to a publicly available uniform resource locator (URL). The primary source for historical data will be clinicaltrials.gov, a public website with extensive records of clinical trial protocols.

As explained above, the agent 120 uses a generated database query to retrieve clinical trial protocol data from a database 140 (see Fig. 1). In some cases, the agent may use a query with a specific National Clinical Trial (NCT) number if, for example, the user request mentions a specific NCT. In such a case, the system produces an API request 210 to be used in an API call to the API 220 of the clinicaltrial.gov repository 230. The API 220 allows for structured data retrieval and augmentation based on various parameters such as study phase, condition, interventions, and outcomes, as shown in the depicted example database response 240.

In embodiments, proprietary data is integrated into this extraction process to enrich the dataset. In such a case, the system may access a proprietary database 250, such as clinical trial data that has been created over the years by companies involved in the setup and management of clinical trials, e.g., Medidata Solutions, Inc., the Applicant of the present application. The query for such access may use all or part of the response 240 from the first, public database 230. In some cases, only the NCT will be used as a query parameter. The result 260 retrieved from the proprietary database 250 may include data not stored in the public database 230, such as trial budget and patient burden index.

The information from both databases can be correlated, e.g., based on NCT number, to produce a more complete response. In embodiments, the correlated response formed from the query of the first, public database 230 and the second, proprietary database 250 may be combined with additional data obtained via API calls performed by the agent 120 to obtain data such as clinical trial metrics, as explained above.

In embodiments, a historical clinical trial protocol database 270 (such as the database 140 depicted in Fig. 1) may be built based on the correlated data of the clinicaltrials.gov database 230 and the proprietary database 250, along with data from other sources. The historical database 270 is constructed as a comprehensive database to house a vast collection of digitized historical clinical trial protocols, as well as the harmonization and ingestion of data into a system designed to support future applications. The database 270 may also integrate various operational metrics from Medidata's extensive proprietary datasets, such as the number of queries, enrollment rates, and other relevant performance metrics. This foundational database 270 provides the raw material needed to train and refine the AI models, ensuring they have access to accurate and extensive historical data from which to learn.

Figs. 3A-3B depict generating a system prompt from a user request and a response obtained when the system prompt is input to a model, such as a large language model (LLM), and subsequent augmentation of the model response using API calls.

The system prompt provides detailed input instructions that guide the LLM in reading, interpreting, and generating the desired output. As explained above, to produce the prompt, the agent 120 combines a user request, e.g., a natural language request, with contextual data to input to the model 130 (see Fig. 1). This contextual data may include various components that aid the model 130 in processing the user request, such as a schema for the database (e.g., database 140). The schema provides a structured framework or blueprint that describes how data is organized and accessed in the database 140, which helps the model 130 to formulate database queries (e.g., SQL queries).

The contextual data may also include domain knowledge definitions. These definitions could involve various elements, such as an electronic data capture (EDC) query definition that specifies the manner of querying data stored in an EDC system, such as Medidata Rave^{®} EDC. Additionally, it might include a patient burden index (PBI) definition which outlines how to interpret PBI data.

Another possible component of the contextual data is an output format definition. This definition specifies the desired structure and format of the data after it has been processed. For example, a valid SQL Dialect (e.g., ANSI SQL) may be specified because it is compatible with particular types of database software. The output format definition may also define the associated metadata (e.g., provided inclusion and/or exclusion criteria and trial phase) to be included in the response from the model.

The contextual data may also include persona instructions for the model. These instructions establish the role the model should take as part of this solution, e.g., the tone and point-of-view the model should adopt in formulating responses to the user requests. For example, the model may adopt the persona of a clinical assistant who is tasked to assist a non-technical person, e.g., a physician, in producing a study protocol.

In the example of Fig. 3A, the user request is: "Provide me with a list of inclusion and exclusion criteria for a Phase 2 study." This results in a system prompt which includes persona instructions ("You are a clinical assistant... "), output format definition ("SQL query in ANSI SQL dialect"), a database schema ("db_schema"), and a metadata section, including tags and instructions for the model ("... extract from the user request the following metadata..."). The system prompt is input to the model to produce a model response, which, in embodiments, includes a database query ("SELECT inclusion_critera FROM eligibility...") and metadata.

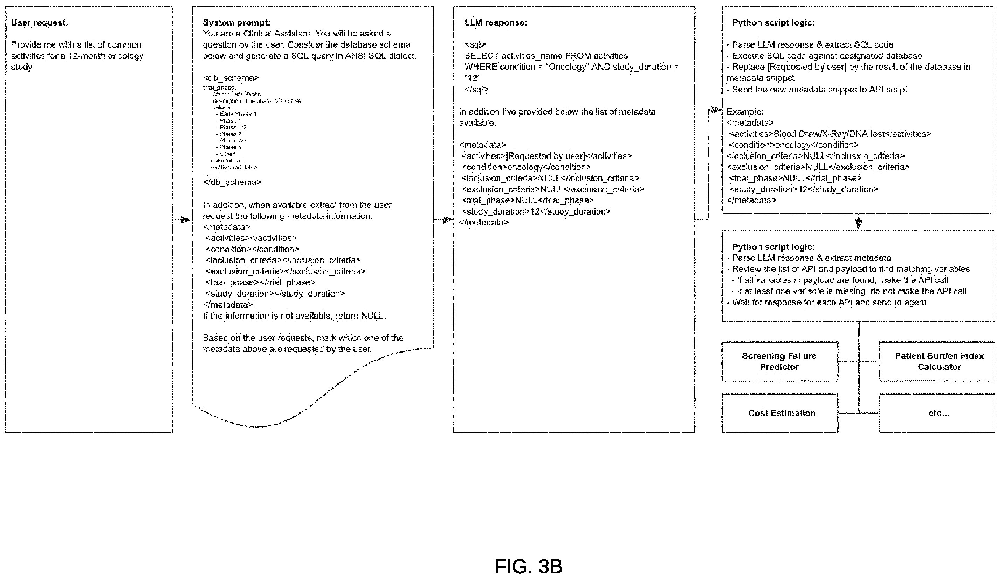

In the example of Fig. 3B, the user request is: "Provide me with a list of common activities for a 12-month oncology study." This results in a system prompt which includes persona instructions ("You are a clinical assistant... "), output format definition ("SQL query in ANSI SQL dialect"), a database schema ("db_schema"), and a metadata section, including tags and instructions for the model ("... extract from the user request the following metadata..."). The system prompt is input to the model to produce a model response, which, in embodiments, includes a database query ("SELECT activities_name FROM activities...") and metadata.

The model response is used to retrieve clinical trial protocol data from a database 140 (see Fig. 1) based at least in part on the database query. In the example of Fig. 3A, the response from the model 130, in this case an LLM response, includes a SQL query and a metadata section corresponding to the metadata section of the user request. Particular tags have been "marked" as requested by the user in the user request, e.g., by inserting a message between the relevant tags, which, in this example, are inclusion_criteria and exclusion_criteria. The indicator "NULL" has been inserted between metadata tags for which metadata has not been obtained from the user request, e.g., activities, condition, and study_duration. Also, based on the user request, the value "2" has been inserted between the tags for trial_phase.

In the example of Fig. 3B, the model response includes a SQL query and a metadata section corresponding to the metadata section of the user request. Particular tags have been "marked" as requested by the user in the user request, e.g., by inserting a message between the relevant tags, which, in this example, are the activities tags. The indicator "NULL" has been inserted between metadata tags for which metadata has not been obtained from the user request, e.g., inclusion_criteria, exclusion_criteria, and trial_phase. Also, based on the user request, the value "12" has been inserted between the tags for study_duration and the term "oncology" has been inserted between the tags for condition.

In embodiments, the retrieval of the clinical trial protocol data from a database may involve parsing the model response to extract the database query and executing the database query against the database. There may also be replacement of at least a portion of the metadata with the retrieved protocol data to produce an augmented model response.

In the example of Fig. 3A, the marker "[Requested by user]" between the inclusion_criteria tags is replaced with "Age is > 18 years old." Also, the placeholder "NULL" between the condition tags is replaced with "oncology." In the example of Fig. 3B, the marker "[Requested by user]" between the activities tags is replaced with "Blood draw/X-ray/DNA test."

The system generates one or more application programming interface (API) requests based at least in part on the retrieved protocol data and/or the metadata. In embodiments, this may involve parsing the augmented model response to extract the metadata and comparing variables of the extracted metadata to variables of the APIs. This may be followed by performing one or more API calls using the generated API requests to obtain clinical trial metrics, such as screening failure prediction, patient burden index, and estimated cost.

In the example of Fig. 3A, the presence of metadata between the condition tags and the trial_phase tags may be sufficient to call the Cost Estimation API. In other cases, calling the Cost Estimation API may also require metadata to be included between the study_duration tags, which means that this API would not be called in the depicted example (although other APIs may be called). In the example of Fig. 3B, the presence of metadata between the activities tags may be sufficient to call the Patient Burden Index API.

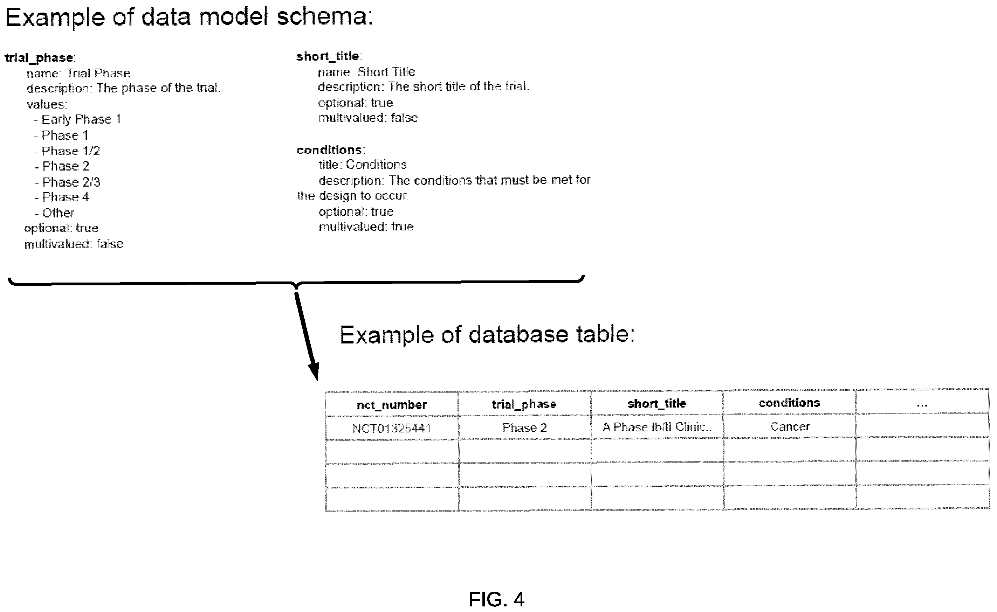

Fig. 4 depicts a data model schema and corresponding database table. In embodiments, the database schema includes tables for storing protocol information, such as trial phases, conditions, interventions, outcomes, demographic data, and other relevant attributes. The schema may be based on the CDISC Unified Study Design Model (USDM) standard, which ensures consistency and compatibility with industry standards. Additionally, as explained above, the data model may be augmented by incorporating proprietary enhancements to better suit specific requirements.

In embodiments, the database is normalized to minimize redundancy and help ensure data integrity. This process involves organizing the data into tables and defining relationships between them to reduce duplication and dependency. Indexing may be implemented to optimize query performance, allowing for quick retrieval of data during the protocol generation process. Indexes are created on key attributes such as protocol identifications (e.g., NCT numbers), study phases, conditions, and interventions. This helps ensure efficient access to the most frequently queried data. Additionally, query optimization techniques are applied to further enhance performance, ensuring that the system can handle large volumes of data with minimal latency.

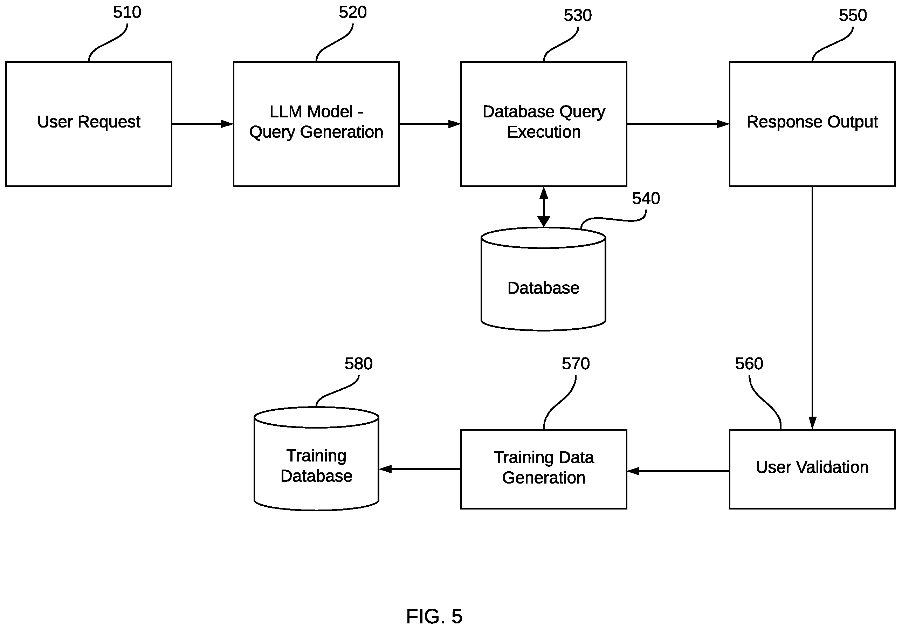

Fig. 5 is a diagram depicting a validation and training system in which user-validated pairs of user requests and database queries are stored in a database to be used for training. Such training is done while the system is in use to improve the accuracy of the system. This is in addition to training done prior to use of the system, in which the output data undergoes a human review process where domain experts will compare the protocols generated based on the system responses against established standards and requirements, providing feedback on the accuracy and relevance of the content.

The system receives a user request 510 which, as explained in the description of Fig. 1, is a natural language statement providing a query and/or instructions for the system to provide results. The LLM model - query generation 520 receives the user request and processes it to generate a database query, e.g., in the form of SQL code. The database query is received by the database query execution 530, which executes the SQL code against the database 540, i.e., retrieves data from the database based on the database query. The LLM model 520 returns a response, which is sent to a response output 550, such as a user interface, to be output to the user.

Once the system is in operation, end-users will have the ability to rate the responses and outputs. Positive ratings from users will indicate high accuracy and relevance, while areas requiring improvement will be identified through lower ratings. As discussed in further detail below, the positively-rated examples are saved and incorporated into a training database to be used in continuously enhancing the performance of the system. This iterative feedback loop ensures that the model evolves and improves over time, maintaining high accuracy and reliability in retrieving data to be used in generating clinical trial protocols.

To generate the training data, the responses from the LLM model 520 are sent for user validation 560, which may be done, for example, via user input at the user interface. The user reviews each response and rates its accuracy by, for example, providing a positive or negative indication or a score. The responses pass to the training data generation 570, which pairs the responses receiving a positive indication from the user with the corresponding user request and stores them in a training database 580. The user request/response pairs, e.g., user request/SQL code pairs, may be used for future model training. Thus, in embodiments, after the outputting of the response, there may be a receiving of a user rating of a response via the user interface and training of the model based at least in part on the user rating.

Multiple approaches, in various combinations, can be taken to train the model 520.

In zero-shot learning, the model 520 leverages pre-trained knowledge to generate output without needing specific examples or a clear definition of "what good looks like." The system prompt can be iteratively refined to provide clearer or more comprehensive information, enhancing the performance of the model. For example, additional schema and formatting information may be included as contextual information in the system prompt. Thus, in embodiments, the inputting of the prompt (e.g., system prompt 320 in Fig. 3A) to the model to produce the model response including the database query (and metadata) may use a zero-shot learning training process and may include iteratively refining the prompt to improve the performance of the model.

In few-shot learning, the model 520 is adapted to specific tasks by training on a small number of examples, which can improve performance. Such examples may be taken from the training database 580 and/or predetermined examples. Thus, in embodiments, the inputting of the prompt to the model to produce the model response including the database query (and metadata) may use a few-shot learning training process and this may include inputting a small number of manually-labeled examples to train the model.

In fine-tuning training, the model 520 is trained on a curated, i.e., user validated, dataset that is continuously updated over time, e.g., the training database 580 described above. The fine-tuning process allows the model to refine its understanding and generate more accurate and relevant outputs based on the evolving dataset. The frequency of the Fine-tuning of the LLM will depend on the availability of the curated dataset.

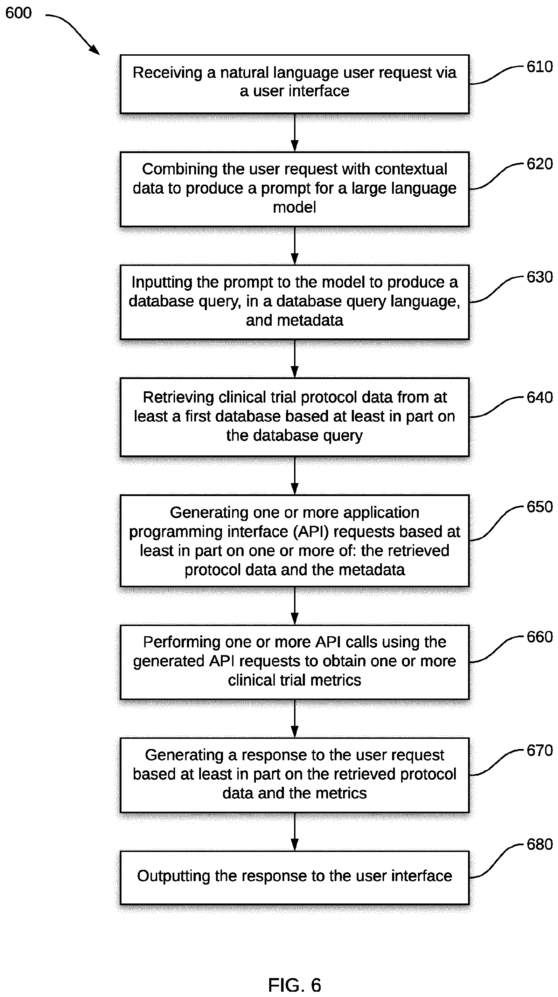

Fig. 6 depicts a method 600 of artificial intelligence - driven clinical trial protocol data retrieval and augmentation, in accordance with disclosed embodiments. The method 600 includes receiving a natural language user request via a user interface (610) and combining the user request with contextual data to produce a prompt for a large language model (620). The method further includes inputting the prompt to the model to produce a model response comprising a database query, in a database query language, and metadata (630) and retrieving clinical trial protocol data from at least a first database based at least in part on the database query (640). The method further includes generating one or more application programming interface (API) requests based at least in part on one or more of: the retrieved protocol data and the metadata (650). The method further includes performing one or more API calls using the generated API requests to obtain one or more clinical trial metrics (660). The method further includes generating a response to the user request based at least in part on the retrieved protocol data and the metrics (670) and outputting the response to the user interface (680).

Aspects of the presently taught approaches may be embodied in the form of a system, a computer program product, or a method. Similarly, aspects of the presently taught approaches may be embodied as hardware, software, or a combination of both. Aspects of the presently taught approaches may be embodied as a computer program product saved on one or more computer-readable media in the form of computer-readable program code embodied thereon.

The computer-readable medium may be a computer-readable storage medium. A computer-readable storage medium may be, for example, an electronic, optical, magnetic, electromagnetic, infrared, or semiconductor system, apparatus, or device, or any combination thereof. A computer-readable storage medium may be described as a non-transitory medium. The computer-readable medium may also or alternatively be a transmission medium by which instructions may be conveyed. A computer-readable transmission medium may include carrier waves, transmission signals or the like. A computer-readable transmission medium may convey instructions between components of a single computer system and/or between plural separate computer systems.

Computer program code in embodiments of the presently taught approaches may be written in any suitable programming and/or scripting language. The program code may execute on a single computer, or on a plurality of computers. The computer may include a processing unit in communication with a computer-usable (computer-readable) medium, where the computer-usable medium contains a set of instructions, and where the processing unit is designed to carry out the set of instructions, and/or a trained machine learning algorithm.

Therefore, from one perspective, there have been described methods and systems for artificial intelligence - driven clinical trial protocol data retrieval and augmentation. A natural language user request is received via a user interface. The user request is combined with contextual data to produce a prompt for a large language model. The prompt is input to the model to produce a model response including a database query, in a database query language, and metadata. Clinical trial protocol data is retrieved from a first database based on the database query. Application programming interface (API) requests are generated based on the database query and/or the metadata. API calls are performed using the generated API requests to obtain clinical trial metrics. A response to the user request is generated based on the retrieved protocol data and the metrics.

Further examples of feature combinations taught by the present disclosure are set out in the following numbered clauses.

Clause 1. A method of artificial intelligence - driven clinical trial protocol data retrieval and augmentation, the method comprising: receiving a natural language user request via a user interface; combining the user request with contextual data to produce a prompt for a large language model; inputting the prompt to the model to produce a model response comprising a database query, in a database query language, and metadata; retrieving clinical trial protocol data from at least a first database based at least in part on the database query; generating one or more application programming interface (API) requests based at least in part on one or more of: the retrieved protocol data and the metadata; performing one or more API calls using the generated API requests to obtain one or more clinical trial metrics; generating a response to the user request based at least in part on the retrieved protocol data and the metrics; and outputting the response to the user interface.

Clause 2. The method of clause 1, wherein, in said combining the user request with the contextual data, the contextual data comprises a schema for the first database.

Clause 3. The method of clause 1 or 2, wherein, in said combining the user request with the contextual data, the contextual data comprises domain knowledge definitions, including one or more of the following: electronic data capture query definition and patient burden index definition.

Clause 4. The method of clause 1, 2 or 3, wherein, in said combining the user request with the contextual data, the contextual data comprises an output format definition.

Clause 5. The method of any preceding clause, wherein, in said combining the user request with the contextual data, the contextual data comprises persona instructions for the model.

Clause 6. The method of any preceding clause, wherein, in said inputting the prompt to the model, the database query language is SQL.

Clause 7. The method of any preceding clause, wherein the first database stores historical clinical trial protocol data and, in said retrieving clinical trial protocol data, the first database is accessed via an API call to a publicly available uniform resource locator (URL).

Clause 8. The method of clause 7, wherein said retrieving clinical trial protocol data further comprises: retrieving additional clinical trial protocol data from a second, proprietary database; and correlating the clinical trial protocol data retrieved from the first database and the second database using respective national clinical trial (NCT) numbers.

Clause 9. The method of any preceding clause, wherein, in said performing said one or more API calls using the generated API requests, said one or more API calls are made to one or more of the following: a screening failure predictor, a budget calculator, and a patient burden index calculator.

Clause 10. The method of any preceding clause, further comprising: parsing the model response to extract the database query; executing the database query, in said retrieving clinical trial protocol data, against said at least first database; and replacing at least a portion of the metadata with the retrieved protocol data to produce an augmented model response.

Clause 11. The method of clause 10, further comprising: parsing the augmented model response to extract the metadata; and comparing, in said generating one or more API requests, variables of the extracted metadata to variables of the APIs.

Clause 12. The method of any preceding clause, wherein said inputting the prompt to the model to produce the model response comprising the database query and metadata uses a zero-shot learning training process.

Clause 13. The method of clause 12, further comprising iteratively refining the prompt to improve the performance of the model.

Clause 14. The method of any preceding clause, wherein said inputting the prompt to the model to produce model response comprising the database query and metadata uses a few-shot learning training process.

Clause 15. The method of clause 14, further comprising inputting a small number of manually-labeled examples to train the model.

Clause 16. The method of any preceding clause, further comprising performing fine tuning of the model using a curated dataset which is continuously updated.

Clause 17. The method of any preceding clause, further comprising: receiving, after said outputting, a user rating of the response via the user interface; and performing training of the model based at least in part on the user rating.

Clause 18. A system for artificial intelligence - driven clinical trial protocol data retrieval and augmentation, the system comprising: a computer having one or more processors in communication with a memory, the memory storing instructions executable by said one or more processors to perform: receiving a natural language user request via a user interface; combining the user request with contextual data to produce a prompt for a large language model; inputting the prompt to the model to produce a model response comprising a database query, in a database query language, and metadata; retrieving clinical trial protocol data from at least a first database based at least in part on the database query; generating one or more application programming interface (API) requests based at least in part on one or more of: the retrieved protocol data and the metadata; performing one or more API calls using the generated API requests to obtain one or more clinical trial metrics; generating a response to the user request based at least in part on the retrieved protocol data and the metrics; and outputting the response to the user interface.

Clause 19. A computer-readable medium comprising instructions that, when executed by one or more processors of a computer, cause said one or more processors to perform a method for artificial intelligence - driven clinical trial protocol data retrieval and augmentation, the method comprising: combining the user request with contextual data to produce a prompt for a large language model; inputting the prompt to the model to produce a model response comprising a database query, in a database query language, and metadata; retrieving clinical trial protocol data from at least a first database based at least in part on the database query; generating one or more application programming interface (API) requests based at least in part on one or more of: the retrieved protocol data and the metadata; performing one or more API calls using the generated API requests to obtain one or more clinical trial metrics; generating a response to the user request based at least in part on the retrieved protocol data and the metrics; and outputting the response to the user interface.

The above discussion is meant to be illustrative of the principles and various embodiments of the presently taught approaches. Numerous variations and modifications will become apparent to those skilled in the art once the above disclosure is fully appreciated. It is intended that the following claims be interpreted to embrace all such variations and modifications.

## Claims

1. A method of artificial intelligence - driven clinical trial protocol data retrieval and augmentation, the method comprising:
receiving a natural language user request via a user interface;
combining the user request with contextual data to produce a prompt for a large language model;
inputting the prompt to the model to produce a model response comprising a database query, in a database query language, and metadata;
retrieving clinical trial protocol data from at least a first database based at least in part on the database query;
generating one or more application programming interface (API) requests based at least in part on one or more of: the retrieved protocol data and the metadata;
performing one or more API calls using the generated API requests to obtain one or more clinical trial metrics;
generating a response to the user request based at least in part on the retrieved protocol data and the metrics; and
outputting the response to the user interface.

2. The method of claim 1, wherein, in said combining the user request with the contextual data, the contextual data comprises a schema for the first database.

3. The method of claim 1 or 2, wherein, in said combining the user request with the contextual data, the contextual data comprises domain knowledge definitions, including one or more of the following: electronic data capture query definition and patient burden index definition.

4. The method of claim 1, 2 or 3, wherein, in said combining the user request with the contextual data, the contextual data comprises an output format definition.

5. The method of any preceding claim, wherein, in said combining the user request with the contextual data, the contextual data comprises persona instructions for the model.

6. The method of any preceding claim, wherein, in said inputting the prompt to the model, the database query language is SQL.

7. The method of any preceding claim, wherein the first database stores historical clinical trial protocol data and, in said retrieving clinical trial protocol data, the first database is accessed via an API call to a publicly available uniform resource locator (URL); and
wherein said retrieving clinical trial protocol data can further comprise: retrieving additional clinical trial protocol data from a second, proprietary database; and correlating the clinical trial protocol data retrieved from the first database and the second database using respective national clinical trial (NCT) numbers.

8. The method of any preceding claim, wherein, in said performing said one or more API calls using the generated API requests, said one or more API calls are made to one or more of the following: a screening failure predictor, a budget calculator, and a patient burden index calculator.

9. The method of any preceding claim, further comprising:
parsing the model response to extract the database query; executing the database query, in said retrieving clinical trial protocol data, against said at least first database; and replacing at least a portion of the metadata with the retrieved protocol data to produce an augmented model response;
and, for example: parsing the augmented model response to extract the metadata; and comparing, in said generating one or more API requests, variables of the extracted metadata to variables of the APIs.

10. The method of any preceding claim, wherein said inputting the prompt to the model to produce the model response comprising the database query and metadata uses a zero-shot learning training process, for example further comprising iteratively refining the prompt to improve the performance of the model.

11. The method of any preceding claim, wherein said inputting the prompt to the model to produce model response comprising the database query and metadata uses a few-shot learning training process, for example further comprising inputting a small number of manually-labeled examples to train the model.

12. The method of any preceding claim, further comprising performing fine tuning of the model using a curated dataset which is continuously updated.

13. The method of any preceding claim, further comprising:
receiving, after said outputting, a user rating of the response via the user interface; and
performing training of the model based at least in part on the user rating.

14. A system for artificial intelligence - driven clinical trial protocol data retrieval and augmentation, the system comprising:
a computer having one or more processors in communication with a memory, the memory storing instructions executable by said one or more processors to perform:
receiving a natural language user request via a user interface;
combining the user request with contextual data to produce a prompt for a large language model;
inputting the prompt to the model to produce a model response comprising a database query, in a database query language, and metadata;
retrieving clinical trial protocol data from at least a first database based at least in part on the database query;
generating one or more application programming interface (API) requests based at least in part on one or more of: the retrieved protocol data and the metadata;
performing one or more API calls using the generated API requests to obtain one or more clinical trial metrics;
generating a response to the user request based at least in part on the retrieved protocol data and the metrics; and
outputting the response to the user interface.

15. A computer-readable medium comprising instructions that, when executed by one or more processors of a computer, cause said one or more processors to perform a method for artificial intelligence - driven clinical trial protocol data retrieval and augmentation, the method comprising:
combining the user request with contextual data to produce a prompt for a large language model;
inputting the prompt to the model to produce a model response comprising a database query, in a database query language, and metadata;
retrieving clinical trial protocol data from at least a first database based at least in part on the database query;
generating one or more application programming interface (API) requests based at least in part on one or more of: the retrieved protocol data and the metadata;
performing one or more API calls using the generated API requests to obtain one or more clinical trial metrics;
generating a response to the user request based at least in part on the retrieved protocol data and the metrics; and
outputting the response to the user interface.
